(19) Europäisches Patentamt
European Patent Office
Office européen des brevets

(11) Publication number: **0 450 892 A1**

# (12) EUROPEAN PATENT APPLICATION

(21) Application number: **91302841.1**

(22) Date of filing: **02.04.91**

(51) Int. Cl.$^5$: **C07C 69/734**, C07C 67/14, A61K 7/42

(30) Priority: **02.04.90 JP 87759/90**
**30.11.90 JP 340795/90**

(43) Date of publication of application:
**09.10.91 Bulletin 91/41**

(84) Designated Contracting States:
**AT BE CH DE DK ES FR GB GR IT LI LU NL SE**

(71) Applicant: **SHISEIDO COMPANY LIMITED**
**7-5-5 Ginza**
**Chuo-ku Tokyo (JP)**

(72) Inventor: **Yoshida, Masashi, c/o Shiseido Laboratories**
**1050, Nippa-cho, Kohoku-ku**
**Yokohama-shi, Kanagawa (JP)**
Inventor: **Umishio, Kenichi, c/o Shiseido Laboratories**
**1050, Nippa-cho, Kohoku-ku**
**Yokohama-shi, Kanagawa (JP)**
Inventor: **Uehara, Keiichi, c/o Shiseido Laboratories**
**1050, Nippa-cho, Kohoku-ku**
**Yokohama-shi, Kanagawa (JP)**

(74) Representative: **Hale, Stephen Geoffrey et al**
**J.Y. & G.W. Johnson Furnival House 14/18 High Holborn**
**London WC1V 6DE (GB)**

## (54) Cinnamic acid derivative and external skin treatment preparation containing the same.

(57) A cinnamic acid derivative having the formula:

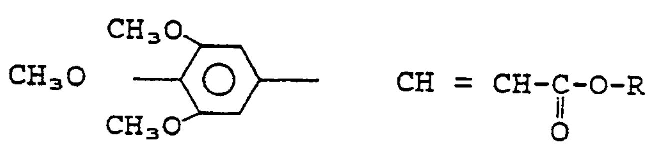

(I)

wherein R represents an alkyl or alkenyl group having 5 to 24 carbon atoms which may contain an oxygen atom and an external skin treatment preparation containing the same.

EP 0 450 892 A1

Jouve, 18, rue Saint-Denis, 75001 PARIS

## BACKGROUND OF THE INVENTION

### 1. Field of the Invention

The present invention relates to a novel cinnamic acid derivative which is soluble in silicone oil, has a high safety factor, a high resistance to water and to oil, and which can absorb UV-rays having a wavelength falling within the UV-B region, as well as an external skin treatment preparation comprising the above-mentioned cinnamic acid derivative and having a suntaninhibitory effect, a satisfactory useability, and an ability to prolong cosmetic effects.

### 2. Description of the Related Art

It is known that UV-rays cause various changes to the skin, and that the UV-rays can be dermatologically classified into long wavelength UV-rays, called UV-A, having a wavelength range of from 400 to 320 nm, medium wavelength UV-rays, called UV-B, having a wavelength range of from 320 to 290 nm, and short wavelength UV-rays, called UV-C, having a wavelength range of 290 nm or shorter.

Generally, most of the UV-rays to which the human body is exposed are sunlight rays, and the UV-rays reaching the earth comprise UV-A and UV-B; the UV-rays falling within the UV-C region are absorbed in the ozone layer and substantially do not reach the earth. Among the UV-rays which reach the earth, the UV-B becomes a cause of various changes to the skin; for example, is a cause of erythema and blistering when the skin is irradiated with the UV-B at a certain dose or higher, further accelerates the formation of melanine, and causes the precipitation of pigment in the skin.

Accordingly, the protection of the skin from irradiation by the UV-B is very important to the prevention of an acceleration of the aging of the skin and a generation or an increase in the splotching or freckling of the skin, and thus a variety of UV-B absorbers have been developed.

Conventionally known UV-B absorbers are, for example, PABA derivatives, cinnamic acid derivatives, salicylic acid derivatives, camphor derivatives, urocanic acid derivatives, benzophenone derivatives and derivatives of heterocyclic compounds, and these UV-B absorbers are exclusively used by incorporation into preparations externally applied to the skin, such as cosmetics and quasi-drugs.

Nevertheless, problems arise in that, since sun-protection cosmetics in which UV-ray absorbers are formulated are in general used in-the hot summer season, they are easily removed by sweating and a secretion of sebaceous matter, and further, when used at the beach or at a swimming pool, are easily removed during bathing. Therefore, there is an increasing demand for a prolonging of the cosmetic effects, and accordingly, a silicone type base such as dimethylsiloxane having a low molecular weight is widely used as a base for externally applied preparations. This is mainly because the silicone type base has required properties such as, for example, a useability such as good spreadability, a good feeling during use, no stickiness, and further, excellent functional characteristics such as a high water resistance and resistance to oil, and a resistance to washing away by sweat and water.

The existing UV-B absorbers, however, have a very low compatibility with the silicone type bases, and therefore, an oily base must be added in addition to the silicone type base when incorporating the silicone type base into preparations for external application, and accordingly, the aforementioned advantages of the silicone type base are not fully realized.

Under the above circumstances, there is now a strong demand for the development of a UV-B absorber which is soluble in silicone oil, has a high water resistance and resistance to oil, and provides a required shield against UV-rays having wavelengths falling within the UV-B region.

## SUMMARY OF THE INVENTION

Accordingly, the objects of the present invention are to eliminate the above-mentioned disadvantages of the prior art and to develop a UV-ray absorber which can be dissolved in silicone oil, has an excellent water resistance and oil resistance, and can provide a satisfactory absorbance of UV-rays having wavelengths in the UV-B region.

Another object of the present invention is to provide a novel cinnamic acid derivative which can be dissolved in silicone oil, has an excellent water resistance and oil resistance, and has an adequate UV-B wavelength blocking region.

A further object of the present invention is to provide an external skin treatment agent having an excellent UV-B absorbing effect and having a UV-B absorber stably formulated therein.

A still further object of the present invention is to provide an external skin treatment agent having an excellent useability or applicability and providing an excellent UV-B absorbing effect by using a silicone base.

Other objects and advantages of the present invention will be apparent from the following description.

In accordance with the present invention, there is provided a cinnamic acid derivative having the formula:

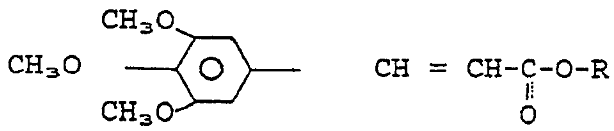

(I)

wherein R represents an alkyl or alkenyl group having 5 to 24 carbon atoms, preferably 6 to 18 carbon atoms, which may contain an oxygen atom.

In accordance with the present invention, there is also provided an external skin treatment preparation which comprises a cinnamic acid derivative having the formula:

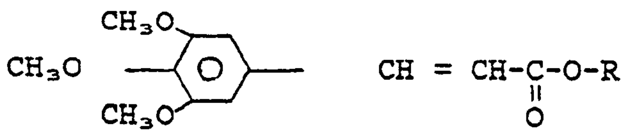

(I)

wherein R represents an alkyl or alkenyl group having 5 to 24 carbon atoms, preferably 6 to 18 carbon atoms, which may contain an oxygen atom.

As a result of intensive studies into this phenomenon, the inventors of this invention found that the cinnamic acid derivatives used in the present invention are compounds which satisfy the foregoing requirements, and thus completed the present invention.

DESCRIPTION OF THE PREFERRED EMBODIMENTS

The present invention will be hereinafter described in detail.

The cinnamic acid derivatives of the present invention can be prepared by subjecting 3,4,5-trimethoxycinnamic acid to an esterification reaction with an alcohol having 5 to 24 carbon atoms.

Examples of R in the formula (I) are alkyl groups having 5 to 24 carbon atoms such as pentyl, hexyl, 3-methylbutyl, neopentyl, 2-ethylbutyl, 1-propylbutyl, octyl, 2-ethylhexyl, 3,5,5-trimethylhexyl, decyl, 3,7-dimethyloctyl, undecyl, tetradecyl, iso-palmityl, iso-stearyl, stearyl, 2-decyltetradecyl, alkenyl groups having 5 to 24 carbon atoms such as 3-methyl-3-butenyl, 11-dodecenyl, palmitoleyl, oleyl, erycyl and oxygen-contains groups such as 2-propoxyethyl, 3-ethoxypropyl, 2-(2-propenyloxy) ethyl, 2-(2-ethoxyethoxy) ethyl.

In the present invention, any bases may be employed if such bases can dissolve the present cinnamic acid derivatives, but in particular, when a silicone type base is used, preparations having desired properties, for example, a good spreadability, a good feeling during use, no stickiness, and further, excellent functional characteristics such as a high water resistance and resistance to oil, and a resistance to washing away by sweat and water, can be obtained.

The silicone type bases usable in the present invention are not specifically restricted, but those usable in the invention include, for example, linear polysiloxanes such as dimethylpolysiloxane, methylpolysiloxane and methylhydrogenpolysiloxane; cyclic polysiloxanes such as decamethylpentasiloxane, dodecamethylhexasiloxane and tetramethyltetrahydrogenpolysiloxane; polyethers, fatty acid-modified polysiloxanes, higher alcohol-modified polysiloxanes and amine-modified polysiloxane.

The external skin treatment preparations according to the present invention may further comprise other components generally used in conventional preparations externally applied to the skin, such as cosmetics and quasi-drugs. For example, it may be possible to optionally add to the preparation, oil components, lubricating oils, ultraviolet-ray absorbers other than those of the present invention, antioxidants, surfactants, preservatives, perfumes, water, alcohols and/or thickening agents.

The fields of application of the external skin treatment preparations according to the present invention are not specifically limited and can be used as cosmetics and quasi-drugs depending on the characteristic properties and purposes of the cinnamic acid derivatives of the present invention.

The external skin treatment preparations according to the present invention can be used in any dosage forms, such as powders, creams, pastes, sticks, liquids, sprays, and a foundation, or can be emulsified in the form of W/O type and O/W type emulsions, with the aid of an emulsifying agent.

The amount of the cinnamic acid derivative to be incorporated may vary, depending on the dosage forms, but preferably is from 0.1 to 20% by weight, more preferably 0.5 to 10% by weight, in the preparation.

The present invention will be hereinafter described in more detail with reference to the following Preparation Examples of novel cinnamic acid derivatives according to the present invention, and the physicochemical properties thereof, but the present invention is not in any way restricted to these specific examples.

Preparation Example 1

First, 96.8 g of 3,4,5-trimethoxycinnamic acid was dispersed in 1 liter of benzene and heated until dissolved, 130 mℓ of thionyl chloride was then added to the solution, and the resulting mixture was stirred at the reflux temperature to form the corresponding acid chloride, followed by the removal of the solvent and the addition of 800 mℓ of toluene containing 36.2 g of 2-pentanol. Then to the mixture was gradually added 500 mℓ of toluene containing triethylamine, the mixture was stirred at room temperature, the reaction mixture was filtered, the resulting solution was washed with water, and the water then removed. The toluene phase was treated with activated charcoal to give 95.6 g (76.2%) of the intended compound, i.e., the 3,4,5-trimethoxycinnamate. The product was further purified by distillation under a reduced pressure.

The resulting product was identified on the basis of the following analytical data.

GC-MS $M^+$ m/z: 308

$^1$H-NMR ($CDCl_3$): 7.58 (1H, CH=CH, d (J=16.1 Hz)); 6.34 (1H, CH=CH, d(J=16.1 Hz)); 6.75 (2H, arom, s); 4.30 (1H, -CH, m); 3.89 (6H, $OCH_3 \times 2$, s); 3.88 (3H, $OCH_3$, s); 1.63 (2H, $CH_2$, q); 1.44 (3H, $CH_3$, d); 1.2-1.4 (2H,$CH_2$, m); 0.92 (3H, $CH_3$, t).

$\lambda_{max}$ (EtOH): 308 nm (ε 19330)

The above UV spectra indicate that the cinnamic acid derivative of the present invention had an excellent ability to absorb UV-rays having wavelengths within the UV-B region.

The water resistance and resistance to oil were determined by mixing the cinnamic acid derivative of the present invention with water, a 50% ethanol solution or an oil such as liquid paraffin, while stirring, and allowing the resulting mixture to stand at 50°C for 60 days to determine whether or not a hydrolysis or the like of the derivative occurred. As a result, no hydrolysis or the like of the derivative of the invention was observed, and thus it was confirmed that the derivative had a high water resistance and resistance to oil.

Preparation Example 2

First, 96.8 g of 3,4,5-trimethoxycinnamic acid was dispersed in 1 liter of benzene and heated until dissolved, 130 mℓ of thionyl chloride was added to the solution, and the resulting mixture was stirred at the reflux temperature to form the corresponding acid chloride, followed by the removal of the solvent and the addition of 800 mℓ of toluene containing 35.4 g of 3-methyl-3-butene-1-ol. Then to the mixture was gradually added 500 mℓ of toluene containing triethylamine, the mixture was stirred at room temperature, the reaction mixture was filtered, the toluene phase was washed with water, and the water then removed. The resulting solution was treated with activated charcoal to give 86.6 g (69.6%) of the intended compound, i.e., the 3,4,5-trimethoxycinnamate. The product was further purified by distillation under a reduced pressure.

The resulting product was identified on the basis of the following analytical data.

GC-MS $M^+$ m/z: 306

$^1$H-NMR ($CDCl_3$): 7.58 (1H, CH=CH, d (J=16.1 Hz)); 6.34 (1H, CH=CH, d(J=16.1 Hz)); 6.75 (2H, arom, s); 4.75 (2H, -$CH_2$, br. s); 4,24 (2H, -$CH_2$-O-, t); 3.89 (6H, $CH_3 \times 2$, s); 3.88 (3H, $OCH_3$, s); 2.54 (2H, $OCH_2$, t); 1.75 (3H, $CH_3$, br. s).

$\lambda_{max}$ (EtOH): 308 nm (ε 19150)

The above UV spectra indicate that the cinnamic acid derivative of the present invention had an excellent ability to absorb UV-rays having wavelengths within the UV-B region.

The water resistance and resistance to oil were determined by mixing the cinnamic acid derivative of the present invention with water, a 50% ethanol solution or an oil such as liquid paraffin, while stirring, and allowing the resulting mixture to stand at 50°C for 60 days to determine whether or not a hydrolysis or the like of the derivative occurred. As a result, no hydrolysis or the like of the derivative of the invention was observed, and thus it was confirmed that the derivative had a high water resistance and resistance to oil.

Preparation Example 3

First, 96.8 g of 3,4,5-trimethoxycinnamic acid was dispersed in 1 liter of benzene and heated until dissolved, 130 mℓ of thionyl chloride was added to the solution, and the resulting mixture was stirred at the reflux temperature to form the corresponding acid chloride, followed by the removal of the solvent and the addition of 800 mℓ of toluene containing 53.5 g of 2-ethylhexyl alcohol. Then to the mixture was gradually added 500 mℓ of toluene containing triethylamine, the mixture was stirred at room temperature, the reaction mixture was filtered, the toluene phase was washed with water, and the water then removed. The resulting solution was treated with activated charcoal to give 81.0 g (56.9%) of the intended compound, i.e., the 3,4,5-trimethoxycinnamate. The product was further purified by distillation under a reduced pressure.

The resulting product was identified on the basis of the following analytical data.

GC-MS: $M^+$ 350

$^1$H-NMR ($CDCl_3$): 7.58 (1H, CH=CH, d (J=16.1 Hz)); 6.34 (1H, CH=CH, d(J=16.1 Hz)); 6.75 (2H, arom, s); 4.13 (2H, -CO-O-$CH_2$-, q); 3.89 (6H, $OCH_3 \times 2$, s); 3.88 (3H, $OCH_3$, s); 1.65 (1H, CH, m); 1.29-1.48 (8H, $CH_2 \times 4$, m); 0.93 (3H, $CH_3$, t); 0.91 (3H, $CH_3$ t).

$\lambda_{max}$ (EtOH): 308 nm (ε 19410)

The above UV spectra indicate that the cinnamic acid derivative of the present invention had an excellent ability to absorb UV-rays having wavelengths within the UV-B region.

The water resistance and resistance to oils were determined by mixing the cinnamic acid derivative of the present invention with water, a 50% ethanol solution or an oil such as liquid paraffin, while stirring, and allowing the resulting mixture to stand at 50°C for 60 days to determine whether or not a hydrolysis or the like of the derivative occurred. As a result, no hydrolysis or the like of the derivative of the invention was observed, and thus it was confirmed that the derivative had a high water resistance and resistance to oil.

Further, the cinnamic acid derivative of the present invetnion was found to have a higher safety feature than that of the mono-methoxy type derivatives, commercially available UV-B absorbers such as 2-ethylhexyl 4-methoxycinnamate.

## Preparation Example 4

First, 96.8 g of 3,4,5-trimethoxycinnamic acid was dispersed in 1 liter of benzene and heated until dissolved, 130 mℓ of thionyl chloride was added to the solution, and the resulting mixture was stirred at the reflux temperature to form the corresponding acid chloride, followed by the removal of the solvent and the addition of 800 mℓ of toluene containing 65.1 g of 1-decanol. Then to the mixture was gradually added 500 mℓ of toluene containing triethylamine, the mixture was stirred at room temperature, the reaction mixture was filtered, the toluene phase was washed with water, and the water then removed. The resulting solution was treated with activated charcoal to give 92.2 g (60.0%) of the intended compound, i.e., the 3,4,5-trimethoxycinnamate. The product was further purified by silica gel column chromatography.

The resulting product was identified on the basis of the following analytical data.

GC-MS $M^+$ m/z: 378

$^1$H-NMR ($CDCl_3$): 7.58 (1H, CH=CH, d (J=16.1 Hz)); 6.34 (1H, CH=CH, d(J=16.1 Hz)); 6.75 (2H, arom, s); 4.10 (2H, -$CH_2$-O-, t); 3.89 (6H, $OCH_3 \times 2$, s); 3.88 (3H, $OCH_3$, s); 1.3-1.6 (16H, $CH_2 \times 8$, m); 0.90 (3H, $CH_3$, t).

$\lambda_{max}$ (EtOH): 308 nm (ε 19040)

The above UV spectra indicate that the cinnamic acid derivative of the present invention had an excellent ability to absorb UV-rays having wavelengths within the UV-B region.

The water resistance and resistance to oils were determined by mixing the cinnamic acid derivative of the present invention with water, a 50% ethanol solution or an oil such as liquid paraffin, while stirring, and allowing the resulting mixture to stand at 50°C for 60 days to determine whether or not a hydrolysis or the like of the derivative occurred. As a result, no hydrolysis or the like of the derivative of the invention was observed, and thus it was confirmed that the derivative had a high water resistance and resistance to oil.

## Preparation Example 5

First, 96.8 g of 3,4,5-trimethoxycinnamic acid was dispersed in 1 liter of benzene and heated until dissolved, 130 mℓ of thionyl chloride was added to the solution, and the resulting mixture was stirred at the reflux temperature to form the corresponding acid chloride, followed by the removal of the solvent and the addition of 800 mℓ of toluene containing 98.6 g of 2-hexyldecanol. Then to the mixture was gradually added 500 mℓ of toluene containing triethylamine, the mixture was stirred at room temperature, the reaction mixture was filtered, the toluene phase was washed with water, and the water then removed. The resulting solution was treated with activated charcoal to give 107.7 g (57.3%) of the intended compound, i.e., the 3,4,5-trimethoxycinnamate. The product was further purified by silica gel column chromatography.

The resulting product was identified on the basis of the following analytical data.

GC-MS $M^+$ m/z: 462

$^1$H-NMR ($CDCl_3$): 7.58 (1H, CH=CH, d (J=16.1 Hz)); 6.34 (1H, CH=CH, d(J=16.1 Hz)); 6.75 (2H, arom, s); 4.11 (2H, $-CH_2-O-$, d); 3.89 (6H, $OCH_3 \times 2$, s); 3.88 (3H, $OCH_3$, s); 1.63 (1H, CH, m); 1.3-1.5 (24H, $CH_2 \times 12$, m); 0.92 (6H, $CH_3 \times 2$, t).

$\lambda_{max}$ (EtOH): 308 nM (ε 19270)

The above UV spectra indicate that the cinnamic acid derivative of the present invention had an excellent ability to absorb UV-rays having wavelengths within the UV-B region.

The water resistance and resistance to oil were determined by mixing the cinnamic acid derivative of the present invention with water, a 50% ethanol solution or an oil such as liquid paraffin, while stirring, and allowing the resulting mixture to stand at 50°C for 60 days to determine whether or not a hydrolysis or the like of the derivative occurred. As a result, no hydrolysis or the like of the derivative of the invention was observed, and thus it was confirmed that the derivative had a high water resistance and resistance to oil.

Preparation Example 6

First, 96.8 g of 3,4,5-trimethoxycinnamic acid was dispersed in 1 liter of benzene and heated until dissolved, 130 mℓ of thionyl chloride was added to the solution, and the resulting mixture was stirred at the reflux temperature to form the corresponding acid chloride, followed by the removal of the solvent and the addition of 800 mℓ of toluene containing 200.9 g of oleyl alcohol. Then to the mixture was gradually added 500 mℓ of toluene containing triethylamine, the mixture was stirred at room temperature, the reaction mixture was filtered, the toluene phase was washed with water, and the water then removed. The resulting solution was treated with activated charcoal to give 121.5 g (61.2%) of the intended compound, i.e., the 3,4,5-trimethoxycinnamate. The product was further purified by silica gel column chromatography.

The resulting product was identified on the basis of the following analytical data.

GC-MS $M^+$ m/z: 488

$^1$H-NMR ($CDCl_3$): 7.58 (1H, CH=CH, d (J=16.1 Hz)); 6.34 (1H, CH=CH, d(J=16.1 Hz)); 6.75 (2H, arom, s); 5.25 (2H, $CH_2-CH=CH-CH_2$ q); 4.09 (2H, $CH_2-O-$, t); 3.89 (6H, $OCH_3 \times 2$, s); 3.88 (3H, $OCH_3$, s); 1.97 (4H, $CH_2 \times 2$, m); 1.3-1.6 (24H, $CH_2 \times 12$, m); 0.88 (3H, CH3, t).

$\lambda_{max}$ (EtOH): 308 nm (ε 19100)

The above UV spectra indicate that the cinnamic acid derivative of the present invention had an excellent ability to absorb UV-rays having wavelengths within the UV-B region.

The water resistance and resistance to oils were determined by mixing the cinnamic acid derivative of the present invention with water, a 50% ethanol solution or an oil such as liquid paraffin, while stirring, and allowing the resulting mixture to stand at 50°C for 60 days to determine whether or not a hydrolysis or the like of the derivative occurred. As a result, no hydrolysis or the like of the derivative of the invention was observed, and thus it was confirmed that the derivative had a high water resistance and resistance to oil.

Further, it was confirmed that the cinnamic acid derivative of the present invention was soluble in silicone type bases.

Moreover, it was also confirmed that the cinnamic acid derivatives obtained in Preparation Examples 1 to 6 were soluble in silicone type bases.

The electronic spectra of UV-ray absorbers are sometimes shifted by 10 to 20 nm, depending on the kinds of bases (or solvents) incorporated. For example, the difference between the electronic spectra of 2-ethylhexyl 4-N,N-dimethylaminobenzoate in ethanol and in dimethylpolysiloxane is 10 nm. Accordingly, there is a strong demand for the development of UV-ray absorbers having a low solvent-dependency. The cinnamic acid derivatives of the present invention show electronic spectra having a low solvent-dependency.

Moreover, the UV-ray absorbers must have a high safety feature and stability against heat and light rays.

The cinnamic acid derivatives according to the present invention do not cause problems concerning safety, such as sensitization, mutagenicity, light-sensitization and phototoxicity, and have a good stability against heat (including a stability against hydrolysis). Regarding a stability against light rays, the derivatives of the present invention are substantially stable against usual sunlight-irradiation, and simply cause a photoisomerization reaction (trans → cis transition) through an irradiation with light from a ultra high pressure mercury lamp, but still absorb UV-rays within the UV-B region. Thus, the derivatives of the present invention are considered to be excellent UV absorbers.

The cinnamic acid derivatives according to the present invention provide a satisfactory shielding against UV-rays within the UV-B region. Further, the cinnamic acid derivatives used in the present invention have a high water resistance and resistance to oil, and therefore, can provide external skin treatment preparations in which the bases and other components can be arbitrarily selected, and at the same time, have an excellent stability even when under severe conditions, for example, under a blazing sun, in the form of anti-suntan cosmetics or the like. The preparations according to the present invention have very useful properties, for example, a good spreadability, a plain feeling during use, no stickiness, and further, excellent functional characteristics such as a high resistance to washing away by sweat and water (an ability to prolong the cosmetic effects), in addition to the foregoing effects, since they comprise a silicone type base. Furthermore, since the UV-B absorber can be incorporated in any amount into preparations to be externally applied to the skin, preparations to be externally applied to the skin can be provided which exhibit any desired shielding effect against UV-rays.

The present invention will further be explained in more detail with reference to the following Working Examples, in which the cinnamic acid derivatives according to the present invention are employed, but the present invention is by no means limited to these specific Examples. In the following Examples, all of the amounts incorporated are expressed in "% by weight", unless otherwise specified.

Example 1: Anti-suntan Cosmetic (oil type)

| | | |
|---|---|---|
| 1. | decamethylcyclopentasiloxane | 48.0% |
| 2. | dimethylpolysiloxane (10CS/25°C) | 20.0 |
| 3. | methylphenylpolysiloxane (20CS/25°C) | 20.0 |
| 4. | silicone resin | 10.0 |
| 5. | 3,4,5-trimethoxycinnamate obtained in Preparation Example 1 | 2.0 |

Comparative Example 1

The same procedures as used in Example 1 were repeated, except that the component 5 in the formulation of Example 1 was omitted, to thus give an oil type anti-suntan cosmetic.

Example 2: Anti-suntan Cosmetic (W/O cream)

| | | |
|---|---|---|
| 1. | octamethylcyclotetrasiloxane | 28.0% |
| 2. | dimethylpolysiloxane (100CS/25°C) | 5.0 |
| 3. | methylphenylpolysiloxane(2,500,000CS/25°C) | 3.0 |
| 4. | liquid paraffin | 5.0 |
| 5. | 3,4,5-trimethoxycinnamate obtained in Preparation Example 6 | 1.5 |
| 6. | polyether-modified silicone (400CS/25°C) (content of polyoxyethylene groups=20% by weight) | 6.0 |
| 7. | purified water | 43.1 |
| 8. | sodium L-glutamate | 3.0 |
| 9. | 1,3-butylene glycol | 5.0 |
| 10. | preservative | 0.2 |
| 11. | perfume | 0.2 |

(Method of the Preparation)

The foregoing components 1 to 6 and 11 were mixed, dissolved by heating, and maintained at 70°C to give an oil phase, and separately, the components 7 to 10 were dissolved by heating and maintained at 70°C to give an aqueous phase. The aqueous phase was then added to the oil phase, and the mixture was thoroughly emulsified with an emulsifier. After the emulsification, the emulsion was cooled, while stirring, poured into a container when the temperature thereof reached 35°C or lower, and allowed to cool to thus solidify the emulsion.

Comparative Example 2

The same procedures as used in Example 2 were repeated, except that the component 5 in the formulation of Example 2 was not used, to thus give a W/O cream type anti-suntan cosmetic.

Example 3: Anti-suntan Cosmetic (O/W cream)

| | | |
|---|---|---|
| 1. | decamethylcyclopentasiloxane | 9.0% |
| 2. | liquid paraffin | 3.0 |
| 3. | isopropyl myristate | 2.0 |
| 4. | vaseline | 5.0 |
| 5. | cetanol | 5.0 |
| 6. | stearic acid | 3.0 |
| 7. | glyceryl monoisostearate | 3.0 |
| 8. | 3,4,5-trimethoxycinnamate obtained in Preparation Example 5 | 0.1 |
| 9. | preservative | 0.2 |
| 10. | perfume | 0.2 |
| 11. | glycerol | 10.0 |
| 12. | propylene glycol | 5.0 |
| 13. | hyaluronic acid | 0.01 |
| 14. | potassium hydroxide | 0.2 |
| 15. | purified water | 54.29 |

(Method of Preparation)

The foregoing components 1 to 10 were heated to 70°C, while stirring, to give an oil phase, and further, the components 11 to 15 were heated to 70°C until completely dissolved, to thus give an aqueous phase. The resulting oil phase was added to the aqueous phase and the mixture was emulsified with an emulsifier. The resulting emulsion was cooled to 30°C by a heat exchanger, and then poured into a container to thus give an O/W cream type anti-suntan cosmetic.

Comparative Example 3

The same procedures as used in Example 3 were repeated, except that the component 8 in the formulation of Example 3 was not used, to thus give an O/W cream type anti-suntan cosmetic.

Example 4: Anti-suntan Lotion

| | | |
|---|---|---|
| 1. | dimethylpolysiloxane (5CS/25°C) | 10.0% |
| 2. | methylphenylpolysiloxane (20CS/25°C) | 7.0 |
| 3. | stearic acid | 1.0 |
| 4. | 3,4,5-trimethoxycinnamate obtained in Preparation Example 3 | 20.0 |
| 5. | preservative | 0.2 |
| 6. | perfume | 0.2 |
| 7. | glycerol | 5.0 |
| 8. | montmorillonite | 0.5 |
| 9. | potassium hydroxide | 0.2 |
| 10. | purified water | 55.9 |

(Method of Preparation)

The foregoing components 1 to 6 were heated to 70°C, while stirring, to give an oil phase, and further, the components 7 to 10 were heated to 70°C until completely dissolved, to thus give an aqueous phase. The resulting oil phase was added to the aqueous phase and the mixture was emulsified with an emulsifier. The resulting emulsion was cooled to 30°C by a heat exchanger and then poured into a container, to thus give an anti-suntan lotion.

Comparative Example 4

The same procedures as used in Example 4 were repeated, except that the component 4 in the formulation of Example 4 was not used, to thus give an anti-suntan lotion.

The UV-ray inhibitory effect of the products prepared in Examples 1 to 4 and Comparative Examples 1 to 4 was determined.

The measurement of the UV-ray inhibitory effect was carried out using the UV-ray-sensitive composition disclosed in Japanese Unexamined Patent Publication (Kokai) No. 62-112020. A method of preparing such a UV-ray-sensitive composition will be described below.

First, a solution (hereinafter referred to as "solution I") was prepared comprising 1.0 g of Leuco Crystal Violet, 1.0 g of tetrabromodimethyl-sulfone, 10 g of an ethylene-vinyl acetate copolymer and 100 mℓ of toluene. Then separately, a solution (hereinafter referred to as "solution II") which comprises 7 g of 2-ethylhexyl 4-N,N-dimethylaminobenzoate, 10 g of an ethylene-vinyl acetate copolymer and 100 mℓ of toluene was prepared.

Then, the solution I was applied onto a base paper for photography, in an amount of 1 g/m$^2$ (solid contents), and dried, and thereafter, the solution II was applied onto the layer of the solution I, in an amount of 5 g/m$^2$ (solid contents), and dried to give a UV-ray-sensitive composition.

The UV-ray-sensitive composition was a paper which undergoes a gradual color change when irradiated with UV-rays; i.e., white → pale purple → purple → dark purple, as the dose of the UV-rays is increased. Each sample to be tested was mixed with 12 g of castor oil and the mixture roll-milled to form a uniform dispersion. A transparent PET film was put on the UV-ray-sensitive composition, which was formed into a circular shape having a diameter of 5 cm, and 1.5 g of the foregoing mixture was applied onto the transparent PET film at a uniform thickness, irradiated with light rays from a UV lamp for 8 minutes, the PET film removed together with the sample layer, and the color difference, by the LAB coordinate system, between the color developed on the UV-ray-sensitive composition and the color thereof observed when the dose of the UV-rays was zero (reference color) was calculated using a 607 Spectrophotometer (available from Hitachi Ltd.)

The results are summarized in the following Table 1.

Table 1

| Sample | Color Difference |
|---|---|
| Example 1 | 25 |
| Comparative Example 1 | 58 |
| Example 2 | 34 |
| Comparative Example 2 | 45 |
| Example 3 | 39 |
| Comparative Example 3 | 57 |
| Example 4 | 30 |
| Comparative Example 4 | 52 |

The results listed in Table 1 indicate that the hues of the Examples each had a color difference smaller than that for the corresponding Comparative Example, and thus had a strong UV-ray inhibitory effect. Namely, an excellent UV-ray inhibitory effect can be ensured by the incorporation of the cinnamic acid derivative, according to the present invention, into the preparations to be externally applied to the skin.

Example 5: Amphibious Anti-suntan Foundation

| | | |
|---|---|---|
| 1. | titanium oxide treated with silicone | 9.5% |
| 2. | mica treated with silicone | 40.0 |
| 3. | talc treated with silicone | 20.45 |
| 4. | iron oxide treated with silicone | 7.5 |
| 5. | spherical nylon powder | 10.0 |
| 6. | trimethylolpropane triisostearate | 5.0 |
| 7. | squalane | 3.0 |
| 8. | bees wax | 2.0 |
| 9. | 3,4,5-trimethoxycinnamate obtained in Preparation Example 2 | 0.5 |
| 10. | sorbitan trioleate | 1.0 |
| 11. | preservative | 0.5 |
| 12. | vitamin E | 0.05 |
| 13. | perfume | 0.5 |

(Method of Preparation)

The foregoing components 1 to 6 were mixed with a Henschel mixer, the components 7 to 13, which had been dissolved by heating and mixed together, were added to and mixed with the foregoing mixture of the components 1 to 6, followed by pulverization of the resulting mixture and molding of the mixture into a medium dish, to thus given an amphibious anti-suntan foundation.

The product of Example 5 was easily spreadable, provided natural results, was capable of prolonging the cosmetic effects, and exhibited a sustained UV-ray inhibitory effect.

Example 6: Anti-suntan Stick Cosmetic

| | | |
|---|---|---|
| 1. | titanium oxide | 10.0% |
| 2. | zinc oxide | 7.0 |
| 3. | mica | 16.0 |
| 4. | red iron oxide | 1.5 |
| 5. | yellow iron oxide | 1.5 |
| 6. | black iron oxide | 1.5 |
| 7. | dimethylpolysiloxane (20CS/25°C) | 29.4 |
| 8. | trimethylolpropane-tri-2-ethylhexanoate | 10.0 |
| 9. | liquid paraffin | 8.0 |
| 10. | microcrystalline wax | 2.0 |
| 11. | ceresine | 1.0 |
| 12. | solid paraffin | 6.0 |
| 13. | 3,4,5-trimethoxycinnamate obtained in Preparation Example 4 | 5.0 |
| 14. | perfume | 0.5 |
| 15. | antioxidant | 0.1 |
| 16. | sorbitan sesquioleate | 1.0 |

(Method of Preparation)

The foregoing components 1 to 6 were mixed with a Henschel mixer, the components 7 to 9, 13, 15 and 16, which had been dissolved with heating and mixed together, were added to and mixed with the foregoing mixture of the components 1 to 6. Then, to the resulting mixer were added the components 10 to 12 and 14, which had been made molten in advance, followed by a thorough mixing of the resulting mixture, and a molding of the mixture into a stick, to thus give an anti-suntan stick cosmetic.

The product of Example 6 had a high UV-ray inhibitory effect and exhibited a prolonged cosmetic effect.

Example 7: Anti-suntan Cosmetic Base

| | | |
|---|---|---|
| 1. | dimethylpolysiloxane (20CS/25°C) | 19.0% |
| 2. | glyceryl triisostearate | 10.0 |
| 3. | Isopar (registered trade mark) G | 5.0 |
| 4. | sorbitan sesquioleate | 1.0 |
| 5. | polyoxyethylene-modified organopolysiloxane | 3.0 |
| 6. | purified water | 46.0 |
| 7. | 1,3-butylene glycol | 5.0 |
| 8. | finely pulverized titanium oxide | 10.0 |
| 9. | 3,4,5-trimethoxycinnamate obtained in Preparation Example 1 | 1.0 |
| 10. | preservative | q.s. |
| 11. | antioxidant | q.s. |
| 12. | perfume | q.s. |

(Method of Preparation)

The foregoing components 1 to 6, 9, 11 and 12 were dissolved at 70°C, while stirring, and the components 7, 8 and 10, which had been heated to 70°C in advance, were added to the resulting solution, followed by an emulsification and dispersion of the resulting mixture, and a cooling thereof, to thus give the intended anti-suntan cosmetic base.

The product of Example 7 had a high UV-ray inhibitory effect and exhibited a prolonged cosmetic effect.

Example 8: Anti-suntan Stick Cosmetic

| | | |
|---|---|---|
| 1. | titanium oxide | 10.0% |
| 2. | zinc oxide | 7.0 |
| 3. | mica | 16.0 |
| 4. | red iron oxide | 1.5 |
| 5. | yellow iron oxide | 1.5 |
| 6. | black iron oxide | 1.0 |
| 7. | dimethylpolysiloxane (20CS/25°C) | 26.4 |
| 8. | trimethylolpropane-tri-2-ethylhexanoate | 8.0 |
| 9. | liquid paraffin | 8.0 |
| 10. | microcrystalline wax | 2.0 |
| 11. | ceresine | 1.0 |
| 12. | solid paraffin | 6.0 |
| 13. | 3,4,5-trimethoxycinnamate obtained in Preparation Example 1 | 10.0 |
| 14. | perfume | 0.5 |
| 15. | antioxidant | 0.1 |
| 16. | sorbitan sesquioleate | 1.0 |

(Method of Preparation)

The foregoing components 1 to 6 were mixed with a Henschel mixer, and the components 7 to 9, 13, 15 and 16, which had been dissolved by heating and mixed together, were added to and mixed with the foregoing mixture of the components 1 to 6. Then, to the resulting mixture were added the components 10 to 12 and 14, which had been made molten in advance, followed by a thorough mixing of the resulting mixture, and molding of the mixture into a stick, to thus give an anti-suntan stick cosmetic.

The product of Example 8 had a UV-ray inhibitory effect higher than that for the product of Example 6, and a further prolonged cosmetic effect.

## Claims

1. A cinnamic acid derivative having the formula:

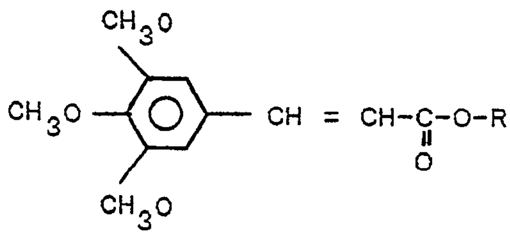

(I)

wherein R represents an alkyl or alkenyl group having 5 to 24 carbon atoms which may contain an oxygen atom.

2. A cinnamic acid derivative as claimed in claim 1, wherein R in the formula (I) represents an alkyl or alkenyl group having 6 to 18 carbon atoms which may contain an oxygen atom.

3. A method of preparing a cinnamic acid derivative having the formula

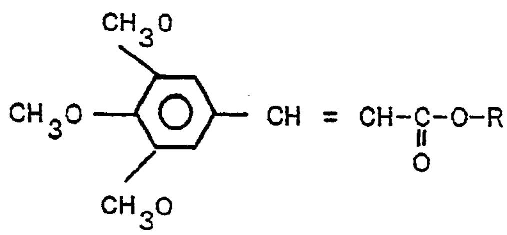

(I)

wherein R represents an alkyl or alkenyl group having 5 to 24 carbon atoms which may contain an oxyqen atom, which method comprises reacting 3, 4, 5-trimethoxycinnamic acid chloride with a compound of the formula ROH, wherein R is as defined above.

4. A skin treatment preparation for topical application comprising:
(i) a cinnamic acid derivative having the formula

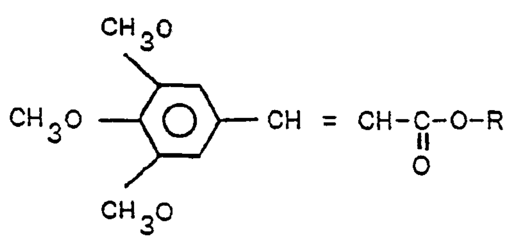

(I)

wherein R represents an alkyl or alkenyl group having 5 to 24 carbon atoms which may contain an oxogen atom and
(ii) a silicone base capable of dissolving the cinnamic acid derivative.

5. A skin treatment preparation as claimed in claim 4, wherein R in the formula (I) represents an alkyl or alkenyl group having 6 to 18 carbon atoms which may contain an oxygen atom.

6. A skin treatment preparation as claimed in claim 4 or 5, wherein the silicone base is at least one component selected from the group consisting of liner polysiloxanes, cyclic polosiloxanes and modified polysiloxanes modified with polyethers, fatty acids, higher alcohols and amines.

7. A skin treatment preparation as claimed in an one of claims 4 to 6, wherein the content of the cinnamic acid derivative is 0.1 to 20% by weight.

8. Use of a cinnamic acid derivative having the formula

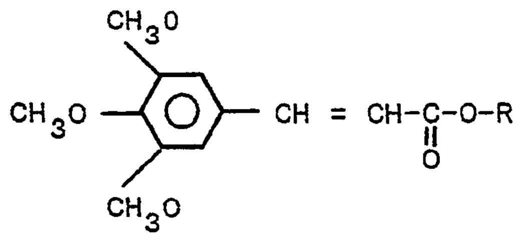

(I)

wherein R represents an alkyl or alkenyl group having 5 to 24 carbon atoms, in the treatment of skin by topical application.

European Patent Office

# EUROPEAN SEARCH REPORT

Application Number EP 91302841.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
| --- | --- | --- | --- |
| X | US - A - 4 709 076 (E. BOMBARDELLI) * Examples 3,5; claims * | 1,3 | C 07 C 69/734<br>C 07 C 67/14<br>A 61 K 7/42 |
| A | EP - A2 - 0 350 314 (SHISEIDO) * Claims * | 4-8 | |
| A | DE - A1 - 3 012 535 (SHISEIDO) * Claims * | 1,3, 4-8 | |

TECHNICAL FIELDS SEARCHED (Int. Cl.5)

C 07 C 69/00
A 61 K 7/00

The present search report has been drawn up for all claims

| Place of search | Date of completion of the search | Examiner |
| --- | --- | --- |
| VIENNA | 17-07-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)